# DEMANDE DE BREVET EUROPEEN

(11) **EP 4 516 282 A1**
(43) Date de publication de la demande: **05.03.2025**
(21) Numéro de dépôt: 24188935.1
(22) Date de dépôt: 16.07.2024
(51) Int. Cl.: A61K 8/49, A61Q 19/02

(54) **NOUVELLES UTILISATIONS DE DÉRIVÉS IMIDAZOLYLS**

(30) Priorité: 01.08.2023 FR 2308332
(71) Demandeur: Exsymol, 98000 Monaco (MC)
(72) Inventeur: VALENTI, Lionel, 06000 Nice (FR)
(74) Mandataire: Murgitroyd & Company

(57) **Abrégé**

Utilisation cosmétique ou dermocosmétique non thérapeutique d'au moins un dérivé imidazolyl de formule générale (I), ou l'un de ses sels cosmétiquement ou dermocosmétiquement acceptables, procédé cosmétique ou dermocosmétique mettant en jeu le susdit au moins un dérivé imidazolyl de formule générale (I), ou l'un de ses sels cosmétiquement ou dermocosmétiquement acceptables, pour réduire localement la visibilité d'une coloration excessive et inesthétique de la peau caractérisée par une accumulation locale de pigments endogènes.

## Description

### Domaine de l'invention

La présente invention concerne de nouvelles utilisations de dérivés imidazolyls pour réduire localement la visibilité d'une manifestation cutanée inesthétique associée à une coloration excessive et inesthétique (et/ou indésirable) de la peau et caractérisée localement par une accumulation de pigments (chromophores) endogènes.

### Etat de la technique

Exemple frappant chez l'Homme de la diversité phénotypique, la peau peut afficher une grande variété de couleurs, pouvant aller de la peau pâle ou presque blanche à des teintes très foncées (noir) en passant par diverses nuances de brun léger et brun marron (Ortonne J.P., Annales de Dermatologie et de Vénéréologie, 2012, vol.139, pp.S73-S77). Indépendamment de facteurs intrinsèques déterminés à la naissance tels le patrimoine génétique, ou encore de facteurs extrinsèques tels l'exposition au soleil, la peau humaine présente une couleur naturelle conférée par un processus biochimique complexe et subtil, celui de la pigmentation. Ainsi au final, la couleur d'une peau dite "normale" chez l'Homme résulte de la contribution et superposition/combinaison de quatre pigments chromophores, que sont précisément l'oxyhémoglobine ou hémoglobine oxygénée (rouge) des vaisseaux capillaires cutanés, l'hémoglobine désoxygénée ou hémoglobine réduite (bleu) des veinules dermiques, le carotène (jaune-orangé) apporté par les caroténoïdes alimentaires, et enfin la mélanine (brun à noir) produite localement dans les mélanocytes, cellules spécialisées de l'épiderme (Casadio S., EMC - Cosmétologie et Dermatologie Esthétique, 2015 - http://dx.doi.org/10.1016/S2211-0380(15)62942-8). Malgré cette pluralité pigmentaire, le facteur prépondérant dans le déterminisme de la couleur de la peau, en conséquence dans les différences et variations de couleur de peau entre les individus, reste le pigment foncé (mélanine), de par notamment sa taille, son intensité et sa typologie, double, avec l'eumélanine (brune à noire foncé) distincte de la phéomélanine (jaune rougeâtre à brun clair), mais également de par le niveau d'activité et de distribution des mélanosomes, organites intracellulaires de synthèse et de stockage des pigments mélaniques avant leur transfert aux kératinocytes afin d'assurer la pigmentation de l'épiderme et sa photoprotection contre l'exposition solaire (Delevoye C. et al., Med. Sci. (Paris), 2011 vol.27(2), pp.153-162). A titre d'illustration, l'on note ainsi qu'une peau à la pigmentation foncée est associée à des mélanosomes plus grands et plus prolifiques, et la mélanine qu'ils produisent est conservée plus longtemps dans les kératinocytes que dans ceux des individus à peau claire (Deng L. et al., Hereditas, 2018, vol.155(1), pp.1-12 et références citées). L'eumélanine est logiquement retrouvée en majorité chez les personnes à la peau foncée, alors que la phéomélanine s'accumule davantage chez les personnes au teint clair (Barsh G.S., PLoS Biology, 2003, vol.1(1), pp.019-022). Enfin, la classification universelle de Fitzpatrick, outil de référence pour les dermatologues, permet de catégoriser les phototypes de peau (6 au total) selon une aptitude, forte à inexistante, du tégument à justement développer une telle pigmentation de couleur foncée à sombre lors d'une exposition solaire (Sachdeva S., Indian J. Dermatol. Venereol. Leprol., 2009, vol.75, pp.93-96).

D'une manière générale et sommaire, les troubles affectant la couleur d'une peau dite "normale" peuvent se résumer à, soit une exagération de la couleur naturelle de la peau avec cliniquement l'apparition localisée de zones pigmentées inégales et notamment de taches plus foncées et sombres, soit à contrario une diminution et pâleur de la peau avec cliniquement l'apparition localisée de taches plus claires (Lipsker D. et al., Annales de Dermatologie et de Vénéréologie, 2019, vol.146(10), pp.666-682). Hormis tout caractère délétère (mélanome, etc.), de tels "défauts de coloration" peuvent d'ailleurs être perçus par l'individu affecté comme inesthétiques et même indésirables car ils sont susceptibles de perturber chez lui l'harmonie du teint escomptée. Dans le but de corriger/estomper de tels défauts affectant l'apparence de la peau (et son côté esthétique) s'est développé le domaine de la dermatologie dite "esthétique et cosmétique" (Wollina U., Dermatologie Therapy, 2008, vol.21 (2), pp.118-130). Au plan lexical, il est acquis par exemple que le substantif "*dyschromie*" qualifie, de manière endogène ou exogène, diffuse ou dans des zones bien circonscrites, tout dépôt dans la peau de pigments divers/quelconques à l'origine d'une modification de la couleur normale de la peau (Braun-Flaco O. et al., Dermatology, 1991, chapter 27). Plus récemment, certains auteurs ont également retenu d'autres attributs génériques pour, identiquement, englober largement toute forme de trouble lié à la décoloration, à la pigmentation et à l'assombrissement de la peau (Nautiyal A. et al., Pigment Cell Melanoma Res., 2021, vol.00, pp.1-15). *De facto,* il est manifeste que l'éventail de tels troubles ayant comme point commun une ou des zones de pigmentation inégale et anormale de la peau, rendant ainsi cette dernière foncée ou décolorée, devient ample car variable en couleurs et en formes. Un tel éventail est ainsi susceptible d'affecter des désordres pigmentaires cutanés de nature :
a) purement dermatologique (concernant des sujets malades (patients), souffrant de ces désordres pigmentaires de nature pathologique), tels les lentigines solaires, le mélasma, la couperose, l'érythème solaire, le vitiligo, mais également
b) esthétique/cosmétique (se produisant notamment chez des sujets « sains », dissociables des sujets malades susvisés), tels l'hyperchromie infra-orbitaire.

Bien que les défauts esthétiques cutanés visés au point b) supra soient tout à fait dissociables des pathologies cutanées visées au point a) supra et concernent généralement des groupes d'individus distincts, tel qu'indiqué précédemment, les solutions cosmétiques (si l'on s'adresse à un sujet présentant les désordres à caractère esthétique/cosmétique visés au point b) supra) et les traitements existants (si l'on s'adresse à un sujet malade (patient), atteint d'une ou plusieurs affection(s) purement dermatologique(s), telle(s) que présentée(s) au point a) supra) ont pour cible la coloration/pigmentation inégale et anormale de la peau affectant la couleur naturelle de la peau. A cet égard, l'état de la technique affiche, depuis une compréhension fondamentale du processus de pigmentation cutanée et de sa complexité, des technologies, des produits et des cibles qualifiés par certains de "cliniquement éprouvés" (Hakozaki T. et al., Textbook of Aging Skin, 2016, pp.1017-1026). Certes, il n'existe pas à proprement parler d'options de référence mais plutôt un arsenal de solutions qui peut aller d'interventions pas ou peu invasives, telles la simple application de produits de maquillage (fond de teint) ou celle plus élaborée de traitements topiques pour éclaircir le teint, ou encore les peelings chimiques, la thérapie au laser, etc., à des manoeuvres chirurgicales et autres interventions de médecine esthétique plus invasives telles la dermabrasion, le microneedling, la greffe de peau, la thérapie cellulaire (Pijpe, A. et al., 2020, pp.109-115). L'une de ces options, prisée de longue date, est en effet l'application locale, avec le support d'une préparation topique, d'ingrédients actifs sur la peau capables d'interférer sur son niveau de pigmentation. A ce titre et notamment dans le cadre d'une pigmentation mélanique excessive, de nombreux ingrédients, d'origine synthétique ou naturelle, ont été développés après-guerre pour leur capacité à éclaircir la peau. Ainsi, les premiers ingrédients historiquement rencontrés dans les crèmes, laits, gels, lotions, sérums et autres formulations cosmétiques ou dermo-cosmétiques à visée blanchissante furent l'hydroquinone (dérivé benzénique) et ses dérivés, des composés mercuriels (oxyde et chlorure de mercure), des rétinoïdes tels l'acide rétinoïque, ou encore certains dermocorticoïdes détournés de leurs usages thérapeutiques. Cependant et en raison de leurs effets néfastes et dangers observés au fil des années sur la santé humaine (potentiellement carcinogènes, problèmes cutanés, etc.), certaines de ces "premières" substances blanchissantes sont aujourd'hui soumises à une réglementation stricte, principalement les législations européenne et américaine, voire, et à l'exemple de l'hydroquinone depuis le règlement (CE) n° 344/2013, apparaissent dans une liste de substances réglementairement interdites dans les produits cosmétiques. Alternativement à ces restrictions et interdictions sanitaires, les fabricants d'ingrédients se sont tournés vers des produits de substitution, soit d'origine naturelle à l'instar de l'acide kojique (produit par plusieurs espèces de champignon), de l'arbutine (hétéroside phénolique contenu dans les feuilles et écorces de nombreuses plantes), de la niacinamide ou de l'acide ascorbique (vitamines naturellement présentes dans certains aliments) ou encore de l'acide azélaïque (issu de la fermentation de graines de céréales ou par lipopéroxydation d'acides gras polyinsaturés via une levure présente à la surface de la peau), soit vers des extraits de plante non cytotoxiques eux aussi doués de propriétés éclaircissantes tels les extraits de réglisse, mûrier et autre busserole (Fernandez X. et al., Techniques de l'ingénieur, 2015, vol. J 2 300, pp.1-33, et références citées). Là encore, certaines réglementations nationales, à l'image de la Suisse, sont ensuite venues restreindre ou interdire l'utilisation de quelques-unes de ces substances d'origine naturelle (acide kojique, arbutine) au regard de la mise en évidence d'effets secondaires indésirables et systémiques : toxicité, irritation, dessèchement et sensibilisation cutanée. Concernant les mécanismes d'action biologique, nombre des ingrédients actifs susmentionnés, mais aussi une multitude d'autres à l'image de ceux compilés dans un récent article de type "review" (Pillaiyar T. et al., J. Med. Chem., 2018, vol.61(17), pp.7395-7418), affichent, en toute logique, un effet inhibiteur sur la tyrosinase, métalloenzyme clef dans la conversion de la tyrosine en mélanine par les mélanocytes, avec un effet partagé entre une action limitante sur la production de mélanine (cas des hydroquinone, acide azélaïque, et arbutine susmentionnés) et celle d'une interaction compétitive avec le cofacteur enzymatique non protéique de la tyrosinase, le cuivre (cas des acides kojique et ascorbique susmentionnés). D'autres agents ont également cherché, entre autres, l'inhibition du transfert des mélanosomes vers les kératinocytes (cas de la niacinamide susmentionnée), l'inhibition de la transcription de la tyrosinase (cas des dérivés de l'acide rétinoïque susmentionné), un blocage de l'inflammation, un piégeage des radicaux libres (cas de l'acide glycyrrhétinique extrait du réglisse susmentionné et de ses racines) mais aussi plus simplement un renouvellement accru de l'épiderme par un effet desquamant afin d'y éliminer l'excès de mélanine (cas des acides alpha-hydroxylés tels les acides glycolique et lactique) (Resende D.I.S.P. et al., Appl. Sci., 2022, vol.12(775), pp.1-15 et références citées).

Ont également étaient décrits, des peptides et composés dits "apparentés", illustrés notamment par des peptides conjugués à des acides carboxyliques (ascorbique, kojique, etc.), tous doués d'une capacité à réduire artificiellement les niveaux de mélanine cutanée en inhibant l'activité enzymatique de la tyrosinase (Boo Y.C., Biomedicines, 2020, vol. 8(9), pp.1-26). Il peut être aussi relevé la synthèse de composés de type benzyl-4(7)-phényl-1H-benzo[d]imidazole 2-substitués, pour leur action inhibitrice sur la tyrosinase à des IC₅₀ proches, pour certains, de la substance de référence, l'acide kojique (Doǧan I.S. et al., Arch. Pharm. Chem. Life Sci., 2016, vol.349(11), pp.881-888), ou encore les demandes de brevet FR2917087 et WO2008006824 ayant respectivement requis la protection de composés de type 4-phenyl-imidazole-2-thiones et imidazole-2-thione, à des fins de prévention, ou de traitement, pharmaceutique ou cosmétique, de troubles pigmentaires, notamment post-inflammatoires ou d'origine métabolique ou médicamenteuse, et ce en dépit du comportement plutôt inducteur de mélanogénèse attendu pour l'histamine et/ou ses sous-produits imidazoles de métabolisation (Tomita Y. et al., J. Dermatol. Sci., 1993, vol. 6(2), pp.146-54 ; Liu J. et al., Skin Pharmacol. Physiol., 2017, vol.30(3), pp.139-145 ; Yoshida M. et al., J. Invest. Dermatol., 2000, vol.114(2), pp.334-42). En outre, les susdits peptides, composés apparentés et dérivés porteurs d'un motif imidazole appartenant à l'art antérieur ne ciblent spécifiquement qu'une action inhibitrice sur l'enzyme tyrosinase et/ou sur la production de pigments mélaniques, ce qui est susceptible de limiter leur efficacité.

En tout état de cause et malgré cette mise à disposition, multiple et variée, de traitements/thérapies destiné(e)s à remédier/corriger ces troubles de la pigmentation susceptibles d'affecter la couleur naturelle de la peau, une telle problématique cutanée continue d'être aujourd'hui, encore et toujours, un « challenge » intéressant aussi bien pour les industries cosmétiques que dermatologiques, car celles-ci sont en recherche perpétuelle de nouveaux ingrédients sûrs et toujours plus performants.

La demande de brevet WO2015/114176 fait état - sous la dénomination commerciale Chronocyclin^{®} - d'un dérivé imidazolyl pour lequel, dans ladite demande, il est décrit les bénéfices suivants sur les cellules de la peau : expression accrue des gènes circadiens CLOCK et PER1, phototransformation activée de la vitamine D; bénéfices différents et distincts de ceux recherchés par la présente invention.

La présente invention s'inscrit pleinement dans le susvisé défi de recherche d'une prise en charge meilleure et efficace visant à remédier, de manière globale et nouvelle/originale, à une manifestation inesthétique ayant comme point commun une ou des zones de coloration excessive(s) - généralement inégale(s) - et inesthétique(s) (et/ou indésirable(s)) de la peau.

### Exposé de l'invention

Aussi, fort de tous ces constats et éléments de littérature, l'inventeur s'est attaché à résoudre le problème technique objectif suivant : mettre à disposition, sur le marché des actifs cosmétiques interagissant sur la production de pigments (chromophores) cutanés, des ingrédients au comportement amélioré et à l'innocuité assurée, avec l'objectif, déterminant à ses yeux, de pouvoir agir concomitamment sur plusieurs composantes bien distinctes de manifestations inesthétiques ayant comme point commun une ou des zones de coloration excessive(s) - généralement inégale(s) - et inesthétique(s) (et/ou indésirable(s)) de la peau, à savoir d'induire une action efficace sur la production de mélanine couplée à celle observée à l'égard d'au moins une autre composante pigmentaire, et leurs facteurs intrinsèques régulateurs impliqués.

Pour ce faire, l'inventeur a établi le cahier des charges suivant :
A) bien que le facteur prépondérant dans le déterminisme de la couleur de la peau reste le pigment foncé (brun/mélanine), tel qu'indiqué précédemment, et qu'une telle pigmentation soit principalement liée à la fonctionnalité des mélanocytes et à l'enzyme (tyrosinase) responsable de la mélanogénèse, tel qu'exposé précédemment, et sans toutefois être liés par la théorie, l'inventeur a eu pour ambition de mettre au point des composés/nouvelles substances ciblant en parallèle d'autres cibles biologiques, à savoir une autre population cellulaire que les seuls mélanocytes et kératinocytes environnants, d'autres facteurs étiologiques intrinsèques susceptibles eux aussi de réguler la pigmentation constitutive de la peau, tels les voies de signalisation produisant le système pigmentaire (Bastonini E. et al., Ann. Dermatol., 2016, vol. 28(3), pp. 279-289) ; cette approche "multifactorielle" :
   - permettant de se démarquer des monothérapies topiques très souvent dirigées vers l'inhibition exclusive de la tyrosinase et production résultante de mélanine, ou encore celle du transfert des mélanosomes chargés en mélanine vers les kératinocytes ; et
   - s'avérant particulièrement adaptée, par exemple, dans le contexte cosmétique (ou dermocosmétique) de l'hyperchromie infra-orbitaire avec l'implication et la mise en exergue d'une dualité de pigments responsables, mélaniques et hémosidérotiques (Souza D.M. et al., Surg. Cosmet. Dermatol., 2011, vol.3(3), pp.233-239) ou encore celui du mélasma et des macules télangiectasiques avec le lien suspecté entre pigmentation et microvascularisation dans la régulation de la pigmentation physiologique de la peau (Regazzetti C. et al., Annales de Dermatologie et de Vénéréologie, 2015, vol.142(12), pp.S482 - Regazzetti C. et al., J. Invest. Dermatol., 2015, vol.135(12), pp.3096-3104) ;
B) ces nouveaux composés/substances destiné(e)s principalement à être incorporé(e)s dans des produits cosmétiques doivent être sanitairement sûr(e)s et conformes aux réglementations européennes cosmétique et chimique (respectivement les règlements (CE) n° 1223/2009 et n° 1907/2006 du Parlement Européen et du Conseil) afin de garantir la sécurité des consommateurs.

La présente invention a donc été développée dans un tel contexte de recherche et cahier des charges - ambitieux - tels qu'indiqués supra. C'est dans ce contexte, qu'à l'issue d'un travail de recherche de grande ampleur, mené notamment à l'aide de différents modèles d'évaluation biologique, l'inventeur a alors découvert, à son étonnement (au regard de l'enseignement de l'état de la technique à date), qu'une famille limitée de composés développés était susceptible d'afficher un comportement général avantageux en respectant les exigences définies aux points A) et B) supra. En l'espèce, il s'agit des dérivés imidazolyls de formule générale (I) suivante (ou l'un de leurs sels cosmétiquement ou dermocosmétiquement acceptables) : 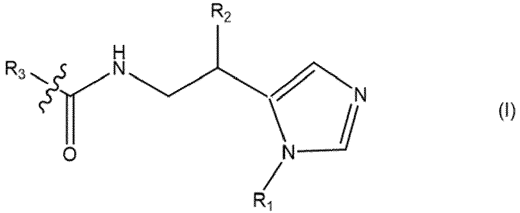 dans laquelle :
- R₁ et R₂ représentent, indépendamment l'un de l'autre, un atome d'hydrogène ou un groupement méthyle ;
- R₃ est :
   i) soit un hétérocycle choisi parmi : 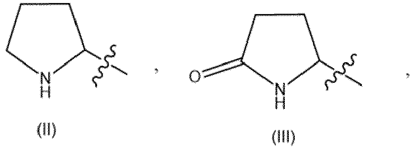
   ii) soit une chaîne alkyle de formule (IV) suivante : 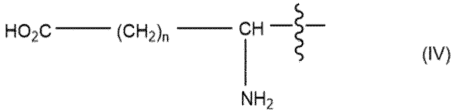 dans laquelle n = 1 ou 2.

L'invention a donc pour objet l'utilisation cosmétique ou dermocosmétique non thérapeutique d'au moins un dérivé imidazolyl de formule générale (I) suivante, ou l'un de ses sels cosmétiquement ou dermocosmétiquement acceptables : 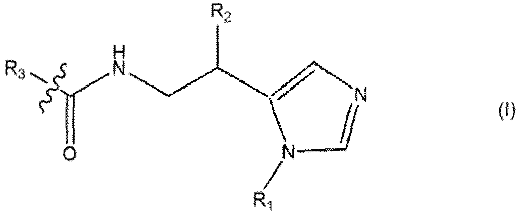 dans laquelle :
- R₁ et R₂ représentent, indépendamment l'un de l'autre, un atome d'hydrogène ou un groupement méthyle ;
- R₃ est :
   i) soit un hétérocycle choisi parmi : 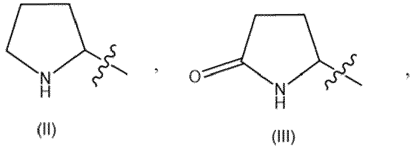
   ii) soit une chaîne alkyle de formule (IV) suivante : 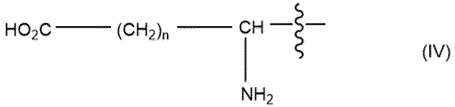 dans laquelle n = 1 ou 2
pour réduire localement la visibilité d'une coloration excessive et inesthétique (et/ou indésirable) de la peau caractérisée par une accumulation locale de pigments endogènes (de préférence responsables de la couleur naturelle de la peau).

Suivant un mode de réalisation préféré de l'invention, l'accumulation locale de pigments endogènes (caractérisant la susdite coloration excessive et inesthétique (et/ou indésirable) de la peau) est une accumulation locale d'au moins deux types de pigments endogènes différents, responsables de la couleur naturelle de la peau, en particulier une accumulation locale de pigments mélaniques et de pigments héminiques.

L'invention a donc également pour objet l'utilisation cosmétique ou dermocosmétique non thérapeutique d'au moins un dérivé imidazolyl de formule générale (I) suivante, ou l'un de ses sels cosmétiquement ou dermocosmétiquement acceptables : 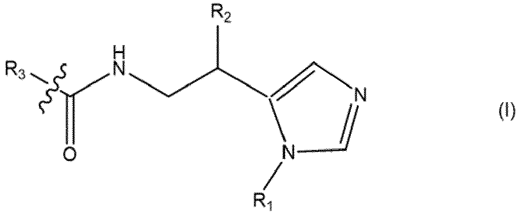 dans laquelle :
- R₁ et R₂ représentent, indépendamment l'un de l'autre, un atome d'hydrogène ou un groupement méthyle ;
- R₃ est :
   i) soit un hétérocycle choisi parmi : 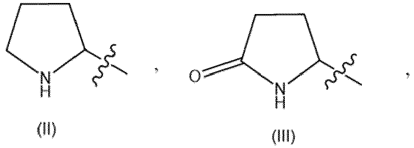
   ii) soit une chaîne alkyle de formule (IV) suivante : 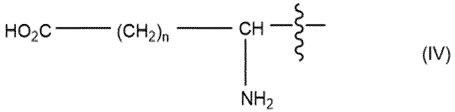 dans laquelle n = 1 ou 2
pour réduire localement la visibilité d'une coloration excessive et inesthétique (et/ou indésirable) de la peau caractérisée par une accumulation locale d'au moins deux types de pigments endogènes différents, responsables de la couleur naturelle de la peau.

Plus précisément, et sans toutefois être liés par la théorie, les composés selon la présente invention possèdent en particulier les caractéristiques suivantes :
- posséder des structures de nature peptidomimétique, en d'autres termes une entité capable de mimer favorablement le comportement biologique de composés peptidiques actifs (oligopeptides, protéines) dans l'espace tridimensionnel, mais avec notamment une stabilité renforcée contre la protéolyse et une biodisponibilité accrue par voie topique ;
- lesdites structures peptidomimétiques étant toutes porteuses d'un noyau aromatique hétérocyclique de type imidazole, de par le couplage peptidique engagé (avec formation d'une liaison de type amide) entre la fonction amine d'une sélection d'amines primaires biogènes contenant un tel motif nucléaire et la fonction acide carboxylique d'une sélection d'acides α-aminés (i.e. aminés en position alpha), ces derniers étant de nature aliphatique ou hétérocyclique.

En outre, comme recherché, ces dérivés imidazolyls de formule générale (I) sont sûrs, du point de vue sanitaire et conformes aux réglementations européennes cosmétique et chimique (respectivement les règlements (CE) n° 1223/2009 et n° 1907/2006 du Parlement Européen et du Conseil) afin de garantir la sécurité des consommateurs.

De surcroît, les dérivés imidazolyls de formule générale (I) utilisables selon l'invention présentent un comportement général avantageux, illustré par un faisceau d'effets techniques bénéfiques, que sont notamment :
- une capacité tout d'abord à piéger et donc bloquer spécifiquement le cofacteur métallique (cuivre) indispensable à l'activité enzymatique de la tyrosinase (cf. exemple 1 ci-après), en conséquence à limiter in fine le fonctionnement biologique de cette dernière, et ce :
   - à une concentration active, dans le cas présent en terme de concentration efficace médiane nécessaire pour chélater la moitié du cuivre Il engagée dans le test de l'exemple 1 (concentration dénommée "ECso"), étonnamment basse, en particulier pour l'un des peptidomimétiques préférés utilisable selon l'invention (67,4 µM soit 0,0014 % ou 14 ppm en concentration massique), et de surcroît au même niveau (65 µM soit 18 ppm en concentration massique) que l'agent dit "de référence" pour la chélation des ions métalliques et la neutralisation de leur toxicité, à savoir l'EDTA ou l'acide éthylènediaminetétraacétique (George T., Brady M.F. Ethylenediaminetetraacetic Acid (EDTA) [Updated 2022 Jun 27]. In: StatPearls [Internet]. Treasure Island (FL): StatPearls Publishing; 2022 Jan-) ;
      - à une susdite ECso (14 ppm) estimée être très différenciante de celle affichée par exemple par des composés peptidiques de l'art antérieur (Yap P.G. et al., Foods, 2021, 10(675), pp. 1-17) présentés comme "anti-tyrosinase" en raison notamment de leur performance dans une même activité de chélation du cuivre Il (protocole expérimental identique à la présente invention), avec effectivement des ECso en agent peptidique chélateur à minima (après extrapolation) de 0,5 % en concentration massique (soit 5000 ppm) et de facto fort éloignées des illustrations selon la présente invention ;
      - à une susdite ECso étonnamment basse pour l'un des peptidomimétiques préférés utilisable selon l'invention (14 ppm), en conséquence une concentration active observée dès la mise en solution de très faibles quantités de soluté, alors qu'à l'opposé les deux substrats [que sont précisément, en nomenclature systématique IUPAC, l'acide (2S)-pyrrolidine-2-carboxylique ou, trivialement, L-proline d'une part, l'amine 2-(1H-imidazol-5-yl)ethanamine ou, trivialement, histamine d'autre part] à l'origine de l'obtention par voie chimique dudit composé préféré utilisable selon l'invention [le (2S)-N-[2-(1H-imidazol-5-yl)ethyl]pyrrolidine-2-carboxamide en nomenclature systématique IUPAC], affichent un comportement chélateur à l'égard d'une même quantité de cuivre Il très en deçà de celui observé pour le peptidomimétique préféré susmentionné, voire même inexistant pour l'un des deux substrats ;
- une capacité ensuite à inhiber l'activité enzymatique de la tyrosinase (cf. exemple 2 ci-après), en conséquence *in fine* à limiter la production spécifique d'une première typologie de pigments (chromophores) responsables de la couleur naturelle de la peau, à savoir les pigments mélaniques, et ce :
   - à une concentration active, dans le cas présent en terme de concentration inhibitrice médiane requise pour atteindre 50% d'inhibition de l'enzyme tyrosinase engagée dans le test de l'exemple 2 (concentration dénommée "*IC₅₀*"):
      - cette dernière estimée être à nouveau différenciante de l'efficacité revendiquée par exemple par un oligopeptide de l'art antérieur commercialement exploité comme agent "anti-mélasma" pour sa capacité à interagir sur l'activité de la tyrosinase et plus largement à ralentir les enzymes clefs du processus de pigmentation (Lima T.N. et al., Cosmetics, 2018, 5(21), pp. 1-9), mais à des concentrations qui apparaissent largement supérieures (à minima de 1 % en concentration massique) au regard du fait rapporté dans ce même article « As a *signal peptide, it interacts well with cells when used at concentrations of 1.0-2.5% »* ;
- une capacité enfin, et c'est un autre aspect majeur, singulier et inattendu de la présente invention, à pouvoir agir en outre sur une composante microvasculaire sous-cutanée à travers précisément, et d'intérêt dans le contexte du point A) ci-dessus évoqué :
   - un effet inhibiteur sur l'expression de molécules protéiques d'adhésion cellulaire vasculaire de type 1 (abréviation "VCAM-1") (cf. exemple 3 ci-après), en d'autres termes des marqueurs présents à la surface de cellules endothéliales en contact direct avec le sang car tapissant l'intérieur des capillaires dermiques :
      - marqueurs dont le rôle est de réguler l'adhésion vasculaire et la diapédèse trans-endothéliale dès lors qu'elles sont associées à un phénomène inflammatoire (Kong D.H. et al., Int. J. Mol. Sciences, 2018, vol.19, pp.1-16) ;
      - en conséquence *in fine,* dans le cadre de la présente invention, une capacité à freiner l'extravasation hors de la vascularisation cutanée d'une seconde typologie de pigments (chromophores) responsables de la couleur naturelle de la peau, celle du chromophore hémoglobine et ses pigments connexes de dégradation (hémosidérine, etc.), eux aussi à l'origine de modifications visibles de la pigmentation dans les couches cutanées et sous-cutanées.

Il est également important de souligner le caractère inattendu des résultats du test objet de l'exemple 3, en particulier de l'effet inhibiteur observé sur l'expression de VCAM-1 au regard du comportement contraire d'une substance dipeptidique structuralement analogue au dérivé imidazolyl de formule générale (I) testé, disponible commercialement, à savoir, en nomenclature systématique IUPAC, l'acide (2*S*)-3-(1*H*-imidazol-5-yl)-2-[[(2*S*)-pyrrolidine-2-carbonyl]amino]propanoïque (dénommé, trivialement, N-L-prolyl-L-histidine).

Il est en outre à souligner que les dérivés imidazolyls de formule générale (I) utilisables selon l'invention sont structurellement éloignés des peptides, composés apparentés et dérivés porteurs d'un motif imidazole appartenant à l'art antérieur et mentionnés supra, à la fin de la section intitulée « Etat de la technique ». D'autre part, ces mêmes peptides, composés apparentés et dérivés porteurs d'un motif imidazole appartenant à l'art antérieur ne ciblent spécifiquement qu'une action inhibitrice sur l'enzyme tyrosinase et/ou sur la production de pigments mélaniques, contrairement aux dérivés imidazolyls de formule générale (I) supra utilisables selon l'invention. Enfin, les résultats affichés par le test de l'exemple 2 de la présente invention sont d'autant plus inattendus qu'il est également à mettre en avant le comportement plutôt inducteur de mélanogénèse exposé pour l'amine biogène histamine et/ou ses sous-produits imidazoles de métabolisation (Tomita Y. et al., J. Dermatol. Sci., 1993, vol. 6(2), pp.146-54).

De préférence, en ce qui concerne l'utilisation selon l'invention, ledit au moins un dérivé imidazolyl de formule générale (I), ou l'un de ses sels cosmétiquement ou dermocosmétiquement acceptables, est :
(a) tel que R₁ et R₂ représentent chacun un atome d'hydrogène, R₃ est un hétérocycle choisi parmi : 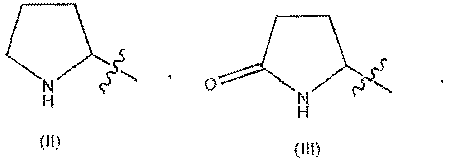 de préférence R₃ étant : 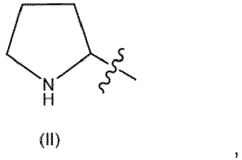 et/ou
(b) tel que R₁ et R₂ représentent chacun un atome d'hydrogène, R₃ est une chaîne alkyle de formule (IV) dans laquelle n est égal à 2 ;
   de manière préférée ledit au moins un dérivé imidazolyl de formule générale (I) étant (en nomenclature systématique IUPAC) :
   - le composé N-[2-(1H-imidazol-5-yl)ethyl]pyrrolidine-2-carboxamide [à savoir, dans la formule générale (I) supra, R₁ et R₂ représentent chacun un atome d'hydrogène, R₃ est de formule (II)], avantageusement son énantiomère de configuration absolue S avec précisément le composé (2S)-N-[2-(1H-imidazol-5-yl)ethyl]pyrrolidine-2-carboxamide, et/ou
   - le composé acide 4-amino-5-[2-(1H-imidazol-5-yl)ethylamino]-5-oxopentanoïque [à savoir, dans la formule générale (I) supra, R₁ et R₂ représentent chacun un atome d'hydrogène, R₃ est une chaîne alkyle de formule (IV) dans laquelle n est égal à 2], avantageusement son énantiomère de configuration absolue S avec précisément le composé acide (4S)-4-amino-5-[2-(1H-imidazol-5-yl)ethylamino]-5-oxopentanoïque.

Les sels desdits dérivés imidazolyls selon l'invention, en particulier des composés préférés susmentionnés, peuvent être obtenus par réaction desdits dérivés et composés avec un acide inorganique, préférentiellement l'acide chlorhydrique, ou un acide organique, en particulier ledit sel cosmétiquement ou dermocosmétiquement acceptable selon l'invention est un chlorhydrate.

Selon un mode de réalisation de l'invention, ladite coloration excessive de la peau caractérisée par l'accumulation locale de pigments endogènes est située au niveau d'une zone cutanée présentant une hyperchromie infra-orbitaire. Dans ce cas, ledit au moins un dérivé imidazolyl de formule générale (I), ou l'un de ses sels cosmétiquement ou dermocosmétiquement acceptables, utilisé aux fins de la présente invention est, de préférence :
(a) tel que R₁ et R₂ représentent chacun un atome d'hydrogène, R₃ est un hétérocycle choisi parmi : 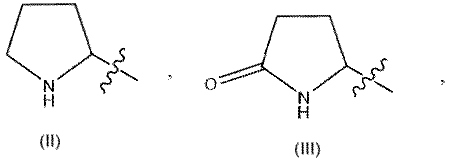 de préférence R₃ étant : 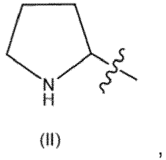 et/ou
(b) tel que R₁ et R₂ représentent chacun un atome d'hydrogène, R₃ est une chaîne alkyle de formule (II) dans laquelle n est égal à 2.

Dans ce mode de réalisation, de manière préférée, ledit au moins un dérivé imidazolyl de formule générale (I) est (en nomenclature systématique IUPAC) :
- le composé N-[2-(1H-imidazol-5-yl)ethyl]pyrrolidine-2-carboxamide [à savoir, dans la formule générale (I) supra, R₁ et R₂ représentent chacun un atome d'hydrogène, R₃ est de formule (II)], avantageusement son énantiomère de configuration absolue S avec précisément le composé (2S)-N-[2-(1H-imidazol-5-yl)ethyl]pyrrolidine-2-carboxamide, et/ou
- le composé acide 4-amino-5-[2-(1H-imidazol-5-yl)ethylamino]-5-oxopentanoïque [à savoir, dans la formule générale (I) supra, R₁ et R₂ représentent chacun un atome d'hydrogène, R₃ est une chaîne alkyle de formule (IV) dans laquelle n est égal à 2], avantageusement son énantiomère de configuration absolue S avec précisément le composé acide (4S)-4-amino-5-[2-(1H-imidazol-5-yl)ethylamino]-5-oxopentanoïque.

Un autre objet de l'invention concerne également l'utilisation susvisée, ledit dérivé imidazolyl de formule générale (I), ou l'un de ses sels cosmétiquement ou dermocosmétiquement acceptables, étant compris dans une composition cosmétique ou dermocosmétique, de préférence dans une teneur de l'ordre de 0,1 à 2% en poids (par exemple comprise entre 0,2 et 1,5% en poids) par rapport au poids total de ladite composition, comprenant en outre au moins un excipient cosmétiquement ou dermocosmétiquement acceptable, de manière préférée ladite composition étant sous une forme adaptée à une administration par voie topique et ledit au moins un excipient étant en outre physiologiquement acceptable avec la peau. En effet, ledit dérivé imidazolyl de formule générale (I) utilisable selon l'invention, ou l'un de ses sels cosmétiquement ou dermocosmétiquement acceptables, utilisable selon l'invention, peut également être mis en oeuvre, à titre d'ingrédient actif cosmétique ou dermocosmétique (par exemple à titre d'unique ingrédient actif cosmétique ou dermocosmétique), dans une composition cosmétique ou dermocosmétique comprenant en outre au moins un excipient cosmétiquement ou dermocosmétiquement acceptable, tel qu'indiqué précédemment. Selon un mode de réalisation, dans un tel contexte de composition cosmétique ou dermocosmétique, ledit dérivé imidazolyl de formule générale (I) est le composé peptidomimétique (2S)-N-[2-(1H-imidazol-5-yl)ethyl]pyrrolidine-2-carboxamide en nomenclature systématique IUPAC [à savoir, dans la formule générale (I) supra, R₁ et R₂ représentent chacun un atome d'hydrogène, R₃ est de formule (II)]. L'invention a également trait à une composition cosmétique ou dermocosmétique non thérapeutique comprenant (ou consistant essentiellement en) au moins un dérivé imidazolyl de formule générale (I) suivante, ou l'un de ses sels cosmétiquement ou dermocosmétiquement acceptables : 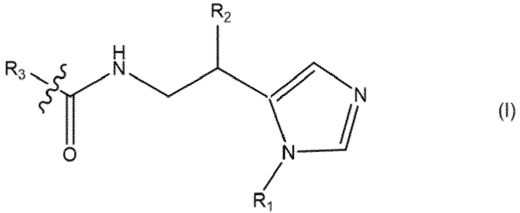 dans laquelle :
- R₁ et R₂ représentent, indépendamment l'un de l'autre, un atome d'hydrogène ou un groupement méthyle ;
- R₃ est :
   i) soit un hétérocycle choisi parmi : 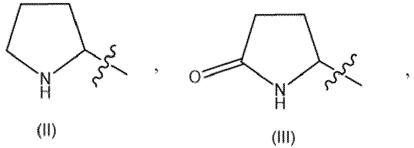
   ii) soit une chaîne alkyle de formule (IV) suivante : 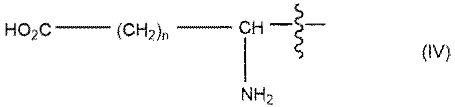 dans laquelle n = 1 ou 2
pour réduire localement la visibilité d'une coloration excessive et inesthétique (et/ou indésirable) de la peau caractérisée par une accumulation locale de pigments endogènes (de préférence responsables de la couleur naturelle de la peau).

Toujours sur le plan « cosmétique » (ou dermocosmétique), l'invention concerne également un procédé cosmétique ou dermocosmétique non thérapeutique pour réduire localement la visibilité d'une coloration excessive et inesthétique (et/ou indésirable) de la peau caractérisée par une accumulation locale d'au moins deux types de pigments endogènes différents, responsables de la couleur naturelle de la peau,
ledit procédé comprenant :
(a) identifier, chez un sujet, une zone de peau présentant ladite coloration excessive et inesthétique (et/ou indésirable) de la peau ; et
(b) administrer audit sujet, par voie topique ou orale, avantageusement par voie topique sur ladite zone de peau, une quantité efficace :
   - d'au moins un dérivé imidazolyl de formule générale (I), ou l'un de ses sels cosmétiquement ou dermocosmétiquement acceptables, tel que défini supra, ou
   - d'une composition cosmétique ou dermocosmétique telle que définie précédemment.

Selon un mode de réalisation, ledit procédé cosmétique ou dermocosmétique non thérapeutique comprend :
(a) identifier, chez un sujet, une zone de peau (saine) présentant ladite coloration excessive et inesthétique (et/ou indésirable) de la peau (par exemple située au niveau d'une zone cutanée présentant une hyperchromie infra-orbitaire) ; et
(b) administrer audit sujet, par voie topique ou orale, avantageusement par voie topique sur ladite zone de peau, une quantité efficace :
   - d'au moins un dérivé imidazolyl de formule générale (I), ou l'un de ses sels cosmétiquement ou dermocosmétiquement acceptables, tel que défini supra, ou
   - d'une composition cosmétique ou dermocosmétique telle que définie précédemment.

Tel qu'indiqué en préambule de la présente demande de brevet un autre aspect de l'invention, distinct de l'aspect « *cosmétique* », concerne l'aspect « *dermatologique* ». A ce titre, l'invention a également pour objet un dérivé imidazolyl de formule générale (I) suivante, ou l'un de ses sels dermatologiquement acceptables : 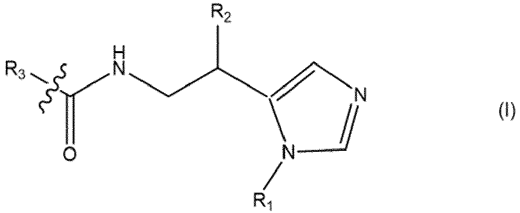 dans laquelle :
- R₁ et R₂ représentent, indépendamment l'un de l'autre, un atome d'hydrogène ou un groupement méthyle ;
- R₃ est :
   i) soit un hétérocycle choisi parmi : 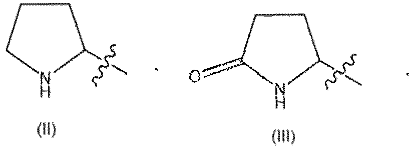
   ii) soit une chaîne alkyle de formule (IV) suivante : 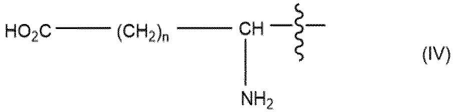 dans laquelle n = 1 ou 2
pour traiter un désordre pigmentaire cutané.

L'invention a également pour objet un dérivé imidazolyl de formule générale (I) suivante, ou l'un de ses sels dermatologiquement acceptables : 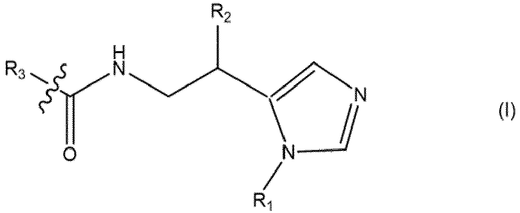 dans laquelle :
- R₁ et R₂ représentent, indépendamment l'un de l'autre, un atome d'hydrogène ou un groupement méthyle ;
- R₃ est :
   i) soit un hétérocycle choisi parmi : 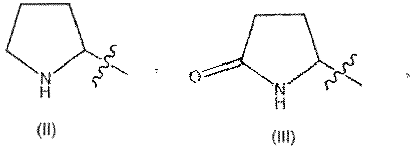
   ii) soit une chaîne alkyle de formule (IV) suivante : 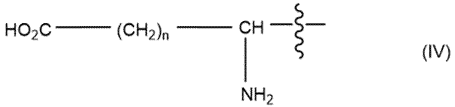 dans laquelle n = 1 ou 2
pour traiter un désordre pigmentaire cutané caractérisé par l'accumulation locale d'au moins deux types de pigments endogènes différents, responsables de la couleur naturelle de la peau.

Selon un mode de réalisation préféré, le susdit au moins un dérivé imidazolyl de formule générale (I) est :
(a) tel que R₁ et R₂ représentent chacun un atome d'hydrogène, R₃ est un hétérocycle choisi parmi : 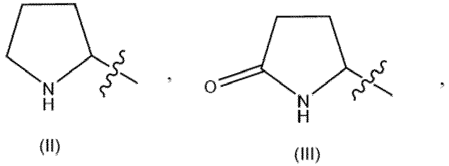 de préférence R₃ étant : 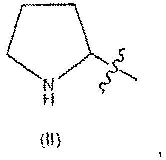 et/ou
(b) tel que R₁ et R₂ représentent chacun un atome d'hydrogène, R₃ est une chaîne alkyle de formule (IV) dans laquelle n est égal à 2 ;
   de manière préférée ledit au moins un dérivé imidazolyl de formule générale (I) étant (en nomenclature systématique IUPAC) :
   - le composé N-[2-(1H-imidazol-5-yl)ethyl]pyrrolidine-2-carboxamide [à savoir, dans la formule générale (I) supra, R₁ et R₂ représentent chacun un atome d'hydrogène, R₃ est de formule (II)], avantageusement son énantiomère de configuration absolue S avec précisément le composé (2S)-N-[2-(1H-imidazol-5-yl)ethyl]pyrrolidine-2-carboxamide, et/ou
   - le composé acide 4-amino-5-[2-(1H-imidazol-5-yl)ethylamino]-5-oxopentanoïque [à savoir, dans la formule générale (I) supra, R₁ et R₂ représentent chacun un atome d'hydrogène, R₃ est une chaîne alkyle de formule (IV) dans laquelle n est égal à 2], avantageusement son énantiomère de configuration absolue S avec précisément le composé acide (4S)-4-amino-5-[2-(1H-imidazol-5-yl)ethylamino]-5-oxopentanoïque.

De préférence, le désordre pigmentaire cutané caractérisé par l'accumulation locale d'au moins deux types de pigments endogènes différents consiste en une accumulation locale de pigments mélaniques et de pigments héminiques.

Selon un mode de réalisation de l'invention, ledit désordre pigmentaire cutané étant sélectionné parmi : les lentigines solaires, le mélasma, la couperose et/ou l'érythème solaire. De préférence il s'agit des lentigines solaires et/ou de la couperose. Dans un tel contexte pour les susdits lentigines solaires et/ou couperose, il est préférentiellement retenu un dérivé imidazolyl de formule générale (I), ou l'un de ses sels dermatologiquement acceptables, dans lequel R₁ et R₂ représentent chacun un atome d'hydrogène, R₃ est un hétérocycle choisi parmi :

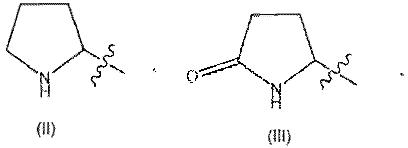

Toujours dans ce même contexte pour les susdits lentigines solaires et/ou couperose, il est tout particulièrement cité, à titre d'exemple non limitatif de dérivé imidazolyl de formule générale (I), le composé peptidomimétique suivant (en nomenclature systématique IUPAC) :

(2S)-N-[2-(1H-imidazol-5-yl)ethyl]pyrrolidine-2-carboxamide.

Par ailleurs, le susdit dérivé imidazolyl de formule générale (I), ou l'un de ses sels dermatologiquement acceptables, pour son utilisation dermatologique, peut être compris dans une préparation dermatologique, de préférence dans une teneur de l'ordre de 0,1 à 2% en poids (par exemple comprise entre 0,2 et 1,5% en poids) par rapport au poids total de ladite préparation, comprenant en outre au moins un excipient dermatologiquement acceptable, de manière préférée ladite préparation étant sous une forme adaptée à une administration par voie topique et ledit au moins un excipient étant en outre physiologiquement acceptable avec la peau. En effet, ledit dérivé imidazolyl de formule générale (I), ou l'un de ses sels dermatologiquement acceptables, utilisable selon l'invention, peut également être mis en oeuvre, à titre d'ingrédient actif dermatologique (par exemple à titre d'unique ingrédient actif dermatologique), dans une préparation dermatologique, cette dernière comprenant en outre au moins un excipient dermatologiquement acceptable, tel qu'indiqué précédemment. Selon un mode de réalisation, dans un tel contexte de préparation dermatologique, ledit dérivé imidazolyl de formule générale (I) est le composé peptidomimétique (en nomenclature systématique IUPAC) : (2S)-N-[2-(1H-imidazol-5-yl)ethyl]pyrrolidine-2-carboxamide.

Les sels desdits dérivés imidazolyls selon l'invention, en particulier des composés préférés susmentionnés, peuvent être obtenus par réaction desdits dérivés et composés avec un acide inorganique, préférentiellement l'acide chlorhydrique, ou un acide organique, en particulier ledit sel dermatologiquement acceptable selon l'invention est un chlorhydrate. Avantageusement, aussi bien ces préparations dermatologiques que les compositions cosmétiques ou dermocosmétiques susvisées sont sous une forme adaptée à une administration par voie topique (cutanée), présentées sous toutes les formes normalement utilisées pour une telle administration. A titre indicatif et non limitatif, elles peuvent se présenter sous la forme d'une solution aqueuse ou hydroalcoolique, éventuellement gélifiée, d'une dispersion du type lotion éventuellement biphasée, d'une émulsion huile-dans-eau ou eau-dans-huile, d'une suspension, d'une lotion, d'une crème, d'un gel aqueux ou hydroalcoolique, ou bien encore sous la forme d'une poudre (micronisée), d'une mousse, d'un sérum, d'une pâte, d'une suspension, d'une dispersion ou d'émulsions multiples pouvant être éventuellement des microémulsions ou des nanoémulsions. En terme d'adjuvants présents dans les compositions, préparations et autres compléments susvisé(e)s et physiologiquement compatibles avec la peau, on peut citer un composé choisi parmi les huiles, les cires, les élastomères de silicone, les tensioactifs, les co-tensioactifs, les épaississants et/ou gélifiants, les humectants, les émollients, les filtres solaires organiques ou inorganiques, les photostabilisants, les conservateurs à l'exception des conservateurs donneurs d'aldéhydes, les colorants, les charges abrasives, les nacres, les agents matifiants, les agents tenseurs, les agents séquestrants, les parfums, etc, et leurs mélanges. A titre indicatif et non limitatif, un tel adjuvant acceptable dans les domaines d'application visés par la présente invention, présent dans la formule dans une teneur de l'ordre de 0,01 à 20% en poids par rapport au poids total de la composition, préparation et autre complément susvisé(e), est notamment cité dans le dictionnaire "International Cosmetic ingrédient Dictionary and Handbook" publié par le "Personnal Care Product Council (PCPC)" de l'Association cosmétique américaine. Enfin, ces mêmes compositions, préparations et autres compléments susvisé(e)s, peuvent comprendre, de manière avantageuse, au moins un ingrédient actif additionnel, l'homme du métier veillant toutefois à ce que ces éventuels actifs additionnels, ainsi que leurs proportions, soient choisis de telle manière que les propriétés avantageuses reconnues à ces compositions, préparations et autres compléments susvisé(e)s ne soient pas, ou substantiellement pas, altéré(e)s par l'adjonction envisagée. De tels actifs additionnels peuvent être choisis, sans que cette liste ne soit limitative, parmi les agents dépigmentants, les agents photoprotecteurs, les agents stimulants la fonction barrière, les agents hydratants ou humectants, les agents desquamants, les agents apaisants, les agents exfoliants, les agents stimulant la prolifération cellulaire tels les fibroblastes et les kératinocytes, les agents déglycants, les agents stimulant la synthèse de collagène ou d'élastine ou prévenant leur dégradation, les agents stimulant la synthèse de glycosaminoglycanes ou de protéoglycanes ou prévenant leur dégradation, les agents antioxydants ou anti-radicalaires ou anti-pollution, les agents stimulant la lipolyse, les agents drainants ou détoxifiants, les agents anti-inflammatoires, les agents accélérateurs de pénétration, les agents desquamants, les agents apaisants et/ou anti-irritants, les agents astringents, les agents exfoliants, les agents agissant sur la microcirculation, etc, et leurs mélanges, un tel ingrédient actif additionnel étant présent dans la formule dans une teneur de l'ordre de 0,0001 à 20% en poids, de préférence de 0,01 à 5% en poids, par rapport au poids total de la composition, préparation et autre complément susvisé(e).

### Définitions

### Définitions générales

Par « *une capacité à freiner l'extravasation hors de la vascularisation cutanée* », il faut comprendre un effet limitant une diffusion/sortie anormale, dans la matrice extra-cellulaire de la peau, de pigments de nature héminique sous l'action d'une inflammation à composante vasculaire.

Par « *en nomenclature systématique IUPAC* », il faut comprendre une dénomination selon un système standardisé des composés chimiques basé sur l'application des recommandations les plus strictes établies par l'Union Internationale de Chimie Pure et Appliquée ("UICPA" en français, ou "IUPAC" en anglais pour "International Union of Pure and Applied Chemistry"), recommandations qui visent à s'assurer qu'un nom correspond à une et une seule structure moléculaire.

Par « *énantiomère de configuration absolue* », il faut comprendre un énantiomère établi selon le système de nomenclature universelle permettant de décrire la configuration absolue de deux énantiomères (système qualifié de "Règles séquentielles de Cahn-Ingold-Prelog").

Par « *énantiomère de configuration absolue S* », il faut comprendre l'énantiomère de configuration dans lequel les substituants prioritaires "tournent" et correspondent à une rotation anti-horaire.

### Définitions inhérentes au domaine cosmétique et dermocosmétique (non thérapeutique)

Par « *hyperchromie infra-orbitaire* », il faut comprendre un désordre à caractère esthétique/cosmétique présentant une ou des zones de pigmentation inégale et anormale de la peau, plus prosaïquement appelé "*cernes colorés*", également retrouvé sous les désignations de "periorbital hyperchromia" (Souza D.M. et al., Surg. Cosmet. Dermatol., 2011, vol.3(3), pp.233-239), ou encore de "*infraorbital discoloration*" (Sarkar R. et al., J. Clin. Aesthet. Dermatol., 2016, vol.9(1), pp.49-55), en d'autres termes l'expression d'un excès de coloration dans la zone du contour de l'œil et qui constitue une autre différence localisée de couleur existante entre la peau palpébrale, fine et transparente, et le reste de la peau du visage (Souza D.M. et al., Surg. Cosmet. Dermatol., 2011, vol.3(3), pp. 233-9). Par ailleurs, il est à souligner que le cerne pigmentaire/foncé dit "*coloré*", étiologiquement distinct des cernes en creux dont l'origine est une disparition avec l'âge de la graisse sous-cutanée péri-oculaire, résulte principalement d'une accumulation tissulaire, autour des yeux, de pigments de nature mélanique et héminique, ces derniers accumulés par l'extravasation du chromophore hémoglobine et ses pigments connexes de dégradation (hémosidérine, etc.) hors de la vascularisation cutanée (Chavan M. et al., J. Cosmet. Sci., 2014, vol.65(2), pp.103-113 ; Vrcek I. et al., J. Cutan. Aesthet. Surg., 2016, vol.9(2), pp.65-72). Ainsi, selon une telle superposition/concentration pigmentaire couplée à l'intensité de cette perméabilité/fuite capillaire, la couleur dudit cerne coloré sera susceptible d'osciller entre une coloration jaunâtre ou brune à noire ou encore bleuâtre-violacée et contribuer ainsi à l'aspect sombre et vieilli de cette zone, fortement inesthétique (et, par conséquent, indésirable).

### Définitions inhérentes au domaine dermatologique

Par « *lentigines solaires* », il faut comprendre une affection de la peau exprimée par des plaques de lésion maculaire, de couleur foncée.

Par « *mélasma* », il faut comprendre encore une affection cutanée relevée principalement chez la femme, notamment lors d'une grossesse avec la prise de traitements hormonaux, et qui se caractérise par l'apparition de plaques irrégulières de lésions brun clair à brun foncé ou gris-brun sur les parties de la peau exposées au soleil, en particulier le visage (Nautiyal A. et al., Pigment Cell Melanoma Res., 2021, vol.00, pp.1-15 et références citées).

Par « *couperose* » ou « *télangiectasie »* selon le terme médical, il faut comprendre une affection de la couleur de la peau lorsqu'elle est associée au développement vasculaire de petits vaisseaux très fins, rouges et parfois même violacés, visibles juste sous la surface de la peau.

### Description détaillée

La description détaillée ci-après a pour but d'exposer l'invention de manière suffisamment claire et complète, notamment à l'aide d'exemples, mais ne doit en aucun cas être regardée comme limitant l'étendue de la protection aux modes de réalisation particuliers et aux exemples présentés ci-après.

### Exemples

### Exemple 1 : test de mise en évidence de la capacité des dérivés imidazolyls utilisables selon l'invention à piéger et bloquer spécifiquement le cofacteur métallique indispensable à l'activité enzymatique de la tyrosinase

Expérimentalement, le test a été réalisé dans les conditions suivantes, basé sur la publication : Seung-hwa Baek et al., "Proton pump inhibitors decrease melanogenesis in melanocytes" Biomedical Reports, 726-730, 2015).

Dans chaque puit d'une micro-plaque de 96 puits, sont placés 100 µL de dérivé imidazolyl utilisable selon l'invention à tester, 50 µL de tampon acétate 200 mM pH 6,0 et 50 µL de solution de CuSO₄ à 800 µM. Après 10 minutes, 50 µL de pyrocatéchol violet (PV) à 800 µM sont ajoutés. Après 20 minutes l'absorbance est mesurée à 632 nm. L'acide éthylènediamine tétraacétique (EDTA, réf. Sigma lot n° MKBQ7974V) est utilisé comme agent de référence (contrôle/témoin positif).

Les résultats sont rassemblés dans le tableau 1 ci-après, notamment exprimés en concentration efficace médiane ou "*EC₅₀*", à savoir une concentration efficace chélatrice de la moitié des ions cuivriques (Cu²⁺) mis en jeu [ECso déterminée expérimentalement en mM puis convertie en % massique à l'aide de la masse molaire moléculaire du dérivé imidazolyl utilisable selon l'invention].

Les dérivés imidazolyls utilisables selon l'invention ont été les composés suivants :
. le (2S)-N-[2-(1H-imidazol-5-yl)ethyl]pyrrolidine-2-carboxamide, à savoir l'énantiomère de configuration absolue S du composé N-[2-(1H-imidazol-5-yl)ethyl]pyrrolidine-2-carboxamide, ce dernier objet de la formule générale (I) supra dans laquelle R₁ = R₂ = H ; R₃ = formule (II) . l'acide (4S)-4-amino-5-[2-(1*H*-imidazol-5-yl)ethylamino]-5-oxopentanoïque, à savoir l'énantiomère de configuration absolue S du composé acide 4-amino-5-[2-(1*H*-imidazol-5-yl)ethylamino]-5-oxopentanoïque, ce dernier objet de la formule générale (I) supra dans laquelle R₁ = R₂ = H ; R₃ = formule (IV) et n = 2

**Tableau 1**

| **Composé (en nomenclature systématique IUPAC)** | **EC₅₀ (µM)** | **EC₅₀ (ppm)** |
|---|---|---|
| EDTA (agent de référence) | 35 | 18 |
| | | (0,0018 % exprimé en % massique) |
| (2S)-N-[2-(1H-imidazol-5-yl)ethyl]pyrrolidine-2-carboxamide [selon l'invention ; lot n° 0098.15] | 37,4 | 14 |
| | | (0,0014 % exprimé en % massique) |
| acide (4S)-4-amino-5-[2-(1*H*-imidazol-5-yl)ethylamino]-5-oxopentanoïque [selon l'invention ; lot n° 5057.22] | 73 | 17 |
| | | (0,0017 % exprimé en % massique) |
| acide (2S)-pyrrolidine-2-carboxylique [substrat ; réf. Sigma lot n° SLBR2808V] | 1400 | 161 |
| | | (0,016 % exprimé en % massique) |
| 2-(1H-imidazol-5-yl)ethanamine [substrat ; réf. Sigma lot n° XWB00664V] | > 10000 | > 1110 |
| | | (> 0,111% exprimé en % massique) |

Les résultats soulignent, pour les dérivés utilisables selon l'invention, une capacité à inhiber le cofacteur métallique indispensable à l'activité enzymatique de la tyrosinase, de surcroît à une concentration active étonnamment faible de l'ordre de celle de l'agent de référence.

### Exemple 2 : test de mise en évidence de la capacité des dérivés imidazolyls utilisables selon l'invention à limiter/inhiber l'activité enzymatique de la tyrosinase

L'évaluation de l'inhibition ou de l'induction de l'activité de la tyrosinase par un dérivé imidazolyl utilisable selon l'invention est effectuée en ajoutant l'enzyme tyrosinase et son substrat, la dopamine, dans des conditions optimales (pH 6,5 et température ambiante, environ 25°C) à une gamme de concentrations du dérivé à tester. Le mélange de test est incubé à température ambiante pendant 15 minutes. Pendant l'incubation, la quantité de production de L-DOPA-quinone dans le mélange réactionnel est déterminée par spectrophotométrie de la formation de dopachrome à 475 nm dans un lecteur de microplaques. L'acide kojique (réf. Sigma K3125 lot n° BCCB7130) est utilisé comme agent de référence (contrôle/témoin positif). Les résultats ont été répétés trois fois pour chaque dérivé imidazolyl utilisable selon l'invention.

Les résultats sont rassemblés dans le tableau 2 ci-après, notamment exprimés en concentration inhibitrice médiane ou "IC₅₀", à savoir une concentration requise pour atteindre une inhibition de 50% de l'activité de la quantité de tyrosinase engagée dans le test [IC₅₀ déterminée expérimentalement en mM puis convertie en % massique à l'aide de la masse molaire moléculaire du dérivé imidazolyl utilisable selon l'invention].

Les dérivés imidazolyls utilisables selon l'invention ont été les composés suivants :
. le (2S)-N-[2-(1H-imidazol-5-yl)ethyl]pyrrolidine-2-carboxamide, à savoir l'énantiomère de configuration absolue S du composé N-[2-(1H-imidazol-5-yl)ethyl]pyrrolidine-2-carboxamide, ce dernier objet de la formule générale (I) supra dans laquelle R₁ = R₂ = H ; R₃ = formule (II) . l'acide (4S)-4-amino-5-[2-(1*H*-imidazol-5-yl)ethylamino]-5-oxopentanoïque, à savoir l'énantiomère de configuration absolue S du composé acide 4-amino-5-[2-(1*H*-imidazol-5-yl)ethylamino]-5-oxopentanoïque, ce dernier objet de la formule générale (I) supra dans laquelle R₁ = R₂ = H ; R₃ = formule (IV) et n = 2

**Tableau 2**

| **Composé (en nomenclature systématique IUPAC)** | **IC₅₀ (mM)** | **IC₅₀ (% massique)** |
|---|---|---|
| acide kojique (agent de référence) | 0,093 | 0,0013 |
| (2S)-N-[2-(1H-imidazol-5-yl)ethyl]pyrrolidine-2-carboxamide [selon l'invention ; lot n° 0098.15] | 1,4 | 0,029 |
| acide (4S)-4-amino-5-[2-(1*H*-imidazol-5-yl)ethylamino]-5-oxopentanoïque [selon l'invention ; lot n° 5057.22] | 2,3 | 0,055 |

Les résultats soulignent, pour les dérivés utilisables selon l'invention, une capacité à inhiber l'activité de la tyrosinase.

### Exemple 3 : test de mise en évidence de la capacité de dérivés imidazolyls utilisables selon l'invention à inhiber l'expression de molécules protéiques d'adhésion cellulaire vasculaire de type 1

Les cellules endothéliales microvasculaires dermiques humaines (HDMEC) ont été obtenues auprès de Promocell (lot 449Z005.3, cellules isolées à partir de tissu mammaire - patiente de 43 ans).

Les cellules ont été cultivées à 37°C et 5% de CO₂ dans le milieu de croissance des cellules endothéliales MV2 + supplément Mix (Promocell). 24 heures après ensemencement, les cellules HDMEC ont été prétraitées avec des dérivés imidazolyls utilisables selon l'invention pendant 2 h avant l'application de 5ng/ml de TNF-alpha (stress). Les cellules ont ensuite été incubées pendant 22 heures.

Après 24 h de traitement, les cellules ont été lavées avec du PBS et un test CELISA a été réalisé pour quantifier l'expression des molécules d'adhésion VCAM-1. Les absorbances ont été mesurées à 450 nm à l'aide d'un spectrophotomètre (Micro-Quant, BioTek Instruments). La déféroxamine (réf. D9533 Sigma lot n° BCCB3792) est utilisée comme agent de référence (contrôle/témoin positif).

Les résultats sont rassemblés dans le tableau 3 ci-après, notamment exprimés en pourcentage d'inhibition sur l'expression de molécules d'adhésion VCAM-1.

Les dérivés imidazolyls utilisables selon l'invention ont été les composés suivants :
. le (2S)-N-[2-(1H-imidazol-5-yl)ethyl]pyrrolidine-2-carboxamide, à savoir l'énantiomère de configuration absolue S du composé N-[2-(1H-imidazol-5-yl)ethyl]pyrrolidine-2-carboxamide, ce dernier objet de la formule générale (I) supra dans laquelle R₁ = R₂ = H ; R₃ = formule (II) . l'acide (4S)-4-amino-5-[2-(1*H*-imidazol-5-yl)ethylamino]-5-oxopentanoïque, à savoir l'énantiomère de configuration absolue S du composé acide 4-amino-5-[2-(1*H*-imidazol-5-yl)ethylamino]-5-oxopentanoïque, ce dernier objet de la formule générale (I) supra dans laquelle R₁ = R₂ = H ; R₃ = formule (IV) et n = 2
. le (2S)-N-[2-(1H-imidazol-5-yl)ethyl]-5-oxopyrrolidine-2-carboxamide, à savoir l'énantiomère de configuration absolue S du composé N-[2-(1H-imidazol-5-yl)ethyl]-5-oxopyrrolidine-2-carboxamide, ce dernier objet de la formule générale (I) supra dans laquelle R₁ = R₂ = H ; R₃ = formule (III)

**Tableau 3**

| **Composé (en nomenclature systématique IUPAC) (concentration)** | **% expression VCAM-1** | **% diminution expression VCAM-1** |
|---|---|---|
| TNF-alpha [contrôle] | 100 | - |
| (5 ng/mL) | | |
| déféroxamine [agent de référence] | 9 | - 91 |
| (50 µM) | | |
| (2S)-N-[2-(1H-imidazol-5-yl)ethyl]pyrrolidine-2-carboxamide [selon l'invention ; lot n° 343.19] | 33 | - 37 |
| (7,5 mM) | | |
| acide (4S)-4-amino-5-[2-(1*H*-imidazol-5-yl)ethylamino]-5-oxopentanoïque [selon l'invention ; lot n° 5057.22] | 73 | - 27 |
| (7,5 mM) | | |
| (2S)-N-[2-(1H-imidazol-5-yl)ethyl]-5-oxopyrrolidine-2-carboxamide [selon l'invention ; lot n° 35476] | 34 | - 36 |
| (7,5 mM) | | |
| acide (2*S*)-3-(1*H*-imidazol-5-yl)-2-[[(2*S*)-pyrrolidine-2-carbonyl]amino]propanoïque [réf. BOC Sciences ; lot n° B22BS12012] | 99 | - 1 |
| (7,5 mM) | | |

Les résultats soulignent, pour les dérivés utilisables selon l'invention, contrairement à, pour l'un d'entre eux, son analogue structural peptidique, une capacité à limiter l'expression de molécules protéiques d'adhésion cellulaire vasculaire de type 1, soit une capacité à freiner l'extravasation hors de la vascularisation cutanée de pigments (chromophores) hémoglobine et pigments connexes de dégradation.

## Revendications

1. Utilisation cosmétique ou dermocosmétique non thérapeutique d'au moins un dérivé imidazolyl de formule générale (I) suivante, ou l'un de ses sels cosmétiquement ou dermocosmétiquement acceptables : 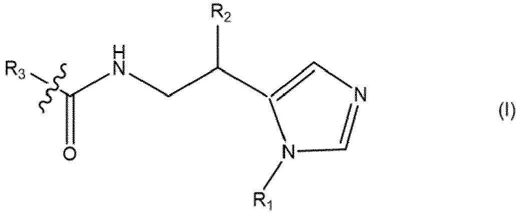 dans laquelle :
- R₁ et R₂ représentent, indépendamment l'un de l'autre, un atome d'hydrogène ou un groupement méthyle ;
- R₃ est :
i) soit un hétérocycle choisi parmi : 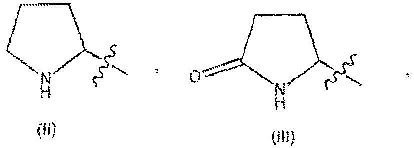
ii) soit une chaîne alkyle de formule (IV) suivante : 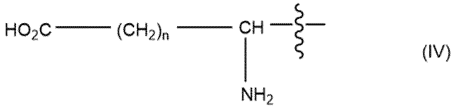 dans laquelle n = 1 ou 2
pour réduire localement la visibilité d'une coloration excessive et inesthétique de la peau chez un sujet sain, **caractérisée par** une accumulation locale d'au moins deux types de pigments endogènes différents, responsables de la couleur naturelle de la peau.

2. Utilisation selon la revendication 1, **caractérisée en ce que** l'accumulation locale d'au moins deux types de pigments endogènes différents, responsables de la couleur naturelle de la peau, est une accumulation locale de pigments mélaniques et de pigments héminiques.

3. Utilisation selon la revendication 1 ou 2, **caractérisée en ce que** ledit au moins un dérivé imidazolyl de formule générale (I) est :
(a) tel que R₁ et R₂ représentent chacun un atome d'hydrogène, R₃ est un hétérocycle choisi parmi : 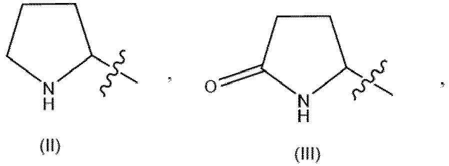 de préférence R₃ étant: 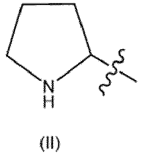 et/ou
(b) tel que R₁ et R₂ représentent chacun un atome d'hydrogène, R₃ est une chaîne alkyle de formule (IV) dans laquelle n est égal à 2 ;
de manière préférée ledit au moins un dérivé imidazolyl de formule générale (I) étant :
- le composé N-[2-(1H-imidazol-5-yl)ethyl]pyrrolidine-2-carboxamide, avantageusement le composé (2S)-N-[2-(1H-imidazol-5-yl)ethyl]pyrrolidine-2-carboxamide, et/ou
- le composé acide 4-amino-5-[2-(1H-imidazol-5-yl)ethylamino]-5-oxopentanoïque, avantageusement le composé acide (4S)-4-amino-5-[2-(1H-imidazol-5-yl)ethylamino]-5-oxopentanoïque.

4. Utilisation selon l'une des revendications précédentes, **caractérisée en ce que** ladite coloration excessive et inesthétique de la peau est située au niveau d'une zone cutanée présentant une hyperchromie infra-orbitaire.

5. Utilisation selon l'une des revendications 1 à 4, ledit dérivé imidazolyl de formule générale (I), ou l'un de ses sels cosmétiquement ou dermocosmétiquement acceptables, étant compris dans une composition cosmétique ou dermocosmétique comprenant en outre au moins un excipient cosmétiquement ou dermocosmétiquement acceptable, de manière préférée ladite composition étant sous une forme adaptée à une administration par voie topique et ledit au moins un excipient étant en outre physiologiquement acceptable avec la peau.

6. Utilisation selon l'une des revendications 1 à 5, ledit sel cosmétiquement ou dermocosmétiquement acceptable dudit dérivé imidazolyl de formule générale (I) étant un chlorhydrate.

7. Dérivé imidazolyl de formule générale (I) suivante, ou l'un de ses sels dermatologiquement acceptables : 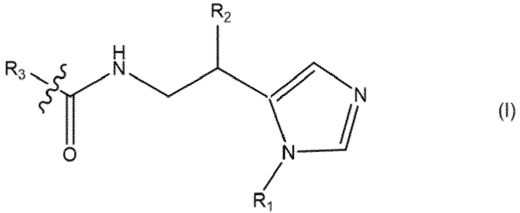 dans laquelle :
- R₁ et R₂ représentent, indépendamment l'un de l'autre, un atome d'hydrogène ou un groupement méthyle ;
- R₃ est :
i) soit un hétérocycle choisi parmi : 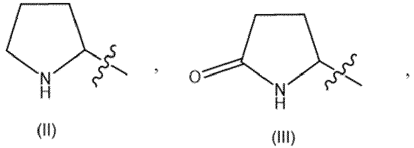
ii) soit une chaîne alkyle de formule (IV) suivante : 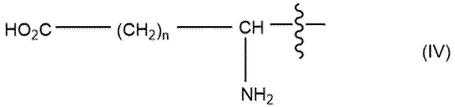 dans laquelle n = 1 ou 2
pour son utilisation dans le traitement d'un désordre pigmentaire cutané **caractérisé par** l'accumulation locale d'au moins deux types de pigments endogènes différents, responsables de la couleur naturelle de la peau.

8. Dérivé imidazolyl de formule générale (I), ou l'un de ses sels dermatologiquement acceptables, selon la revendication 7, pour son utilisation selon la revendication 7, dans lequel l'accumulation locale d'au moins deux types de pigments endogènes différents, responsables de la couleur naturelle de la peau, est une accumulation locale de pigments mélaniques et de pigments héminiques.

9. Dérivé imidazolyl de formule générale (I), ou l'un de ses sels dermatologiquement acceptables, selon la revendication 7 ou 8, pour son utilisation selon la revendication 7 ou 8, ledit désordre pigmentaire cutané étant sélectionné parmi les lentigines solaires, le mélasma, la couperose et/ou l'érythème solaire.

10. Dérivé imidazolyl de formule générale (I), ou l'un de ses sels dermatologiquement acceptables, selon l'une des revendications 7 à 9, pour son utilisation selon l'une des revendications 7 à 9, **caractérisé en ce que** ledit au moins un dérivé imidazolyl de formule générale (I) est :
(a) tel que R₁ et R₂ représentent chacun un atome d'hydrogène, R₃ est un hétérocycle choisi parmi : 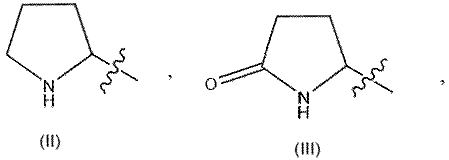 de préférence R₃ étant : 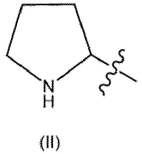 et/ou
(b) tel que R₁ et R₂ représentent chacun un atome d'hydrogène, R₃ est une chaîne alkyle de formule (IV) dans laquelle n est égal à 2 ;
de manière préférée ledit au moins un dérivé imidazolyl de formule générale (I) étant :
- le composé N-[2-(1H-imidazol-5-yl)ethyl]pyrrolidine-2-carboxamide, avantageusement le composé (2S)-N-[2-(1H-imidazol-5-yl)ethyl]pyrrolidine-2-carboxamide, et/ou
- le composé acide 4-amino-5-[2-(1H-imidazol-5-yl)ethylamino]-5-oxopentanoïque, avantageusement le composé acide (4S)-4-amino-5-[2-(1H-imidazol-5-yl)ethylamino]-5-oxopentanoïque.

11. Dérivé imidazolyl de formule générale (I), ou l'un de ses sels dermatologiquement acceptables, selon l'une des revendications 7 à 10, pour son utilisation selon l'une des revendications 7 à 10, ledit dérivé imidazolyl de formule générale (I), ou l'un de ses sels dermatologiquement acceptables, étant compris dans une préparation dermatologique comprenant en outre au moins un excipient dermatologiquement acceptable, de manière préférée ladite préparation étant sous une forme adaptée à une administration par voie topique et ledit au moins un excipient étant en outre physiologiquement acceptable avec la peau.

12. Dérivé imidazolyl de formule générale (I), ou l'un de ses sels dermatologiquement acceptables, selon l'une des revendications 7 à 11, pour son utilisation selon l'une des revendications 7 à 11, ledit sel dermatologiquement acceptable dudit dérivé imidazolyl de formule générale (I) étant un chlorhydrate.

13. Utilisation selon la revendication 5, ou dérivé imidazolyl de formule générale (I), ou l'un de ses sels dermatologiquement acceptables, selon la revendication 11, pour son utilisation selon la revendication 11, ladite composition cosmétique ou dermocosmétique telle que définie au sein de la revendication 5 ou ladite préparation dermatologique telle que définie au sein de la revendication 11 comprenant en outre au moins un ingrédient actif additionnel choisi parmi les agents dépigmentants, les agents photoprotecteurs, les agents stimulants la fonction barrière, les agents hydratants ou humectants, les agents desquamants, les agents apaisants, les agents exfoliants, les agents stimulant la prolifération cellulaires tels les fibroblastes et les kératinocytes, les agents déglycants, les agents stimulant la synthèse de collagène ou d'élastine ou prévenant leur dégradation, les agents stimulant la synthèse de glycosaminoglycanes ou de protéoglycanes ou prévenant leur dégradation, les agents antioxydants ou anti-radicalaires ou anti-pollution, les agents stimulant la lipolyse, les agents drainants ou détoxifiants, les agents anti-inflammatoires, les agents accélérateurs de pénétration et/ou anti-irritants, les agents astringents, les agents agissant sur la microcirculation, et leurs mélanges.

14. Procédé cosmétique ou dermocosmétique non thérapeutique pour réduire localement la visibilité d'une coloration excessive et inesthétique de la peau **caractérisée par** une accumulation locale d'au moins deux types de pigments endogènes différents, responsables de la couleur naturelle de la peau,
ledit procédé comprenant :
(a) identifier, chez un sujet sain, une zone de peau présentant ladite coloration excessive et inesthétique de la peau ; et
(b) administrer audit sujet, par voie topique ou orale, avantageusement par voie topique sur ladite zone de peau, une quantité efficace :
- d'au moins un dérivé imidazolyl de formule générale (I), ou l'un de ses sels cosmétiquement ou dermocosmétiquement acceptables, tel que défini au sein de la revendication 1 ou 3, ou
- d'une composition cosmétique ou dermocosmétique telle que définie au sein de la revendication 5.
